# EUROPEAN PATENT APPLICATION

(11) **EP 1 955 708 A1**
(43) Date of publication of application: **13.08.2008**
(21) Application number: 06832989.5
(22) Date of filing: 21.11.2006
(51) Int. Cl.: A61K 45/06, A61K 31/175, A61K 31/4745, A61K 31/675, A61K 31/7084, A61K 31/7088, A61K 38/00, A61K 39/00, A61K 39/395, A61P 31/12, A61P 35/00, A61P 37/04

(54) **NOVEL MEMORY CTL INDUCTION POTENTIATOR**

(30) Priority: 24.11.2005 JP 2005337990
(71) Applicant: Dainippon Sumitomo Pharma Co., Ltd., Osaka 541-8524 (JP)
(72) Inventor: TOMIZAWA, Hideyuki, Osaka-shi Osaka 554-0022 (JP)
(74) Representative: Duckworth, Timothy John
(86) International application number: PCT/JP2006/323139
(87) International publication number: WO 2007/060918

(57) **Abstract**

The present invention relates to a memory CTL induction enhancer comprising a combination of substance (b) with substance (a) and/or substance (c), wherein substance (a) is an activator of antigen-presenting cells, substance (b) is an inducer of homeostatic proliferation after induction of lymphopenia, and substance (c) is a suppressor of regulatory T cells.

## Description

### Technical Field

The present invention relates to a novel memory CTL induction enhancer. More specifically, the present invention relates to a memory CTL induction enhancer comprising a combination of substance (b) with substance (a) and/or substance (c), wherein substance (a) is an activator of antigen-presenting cells, substance (b) is an inducer of homeostatic proliferation after induction of lymphopenia, and substance (c) is a suppressor of regulatory T cells.

### Background Art

The history of vaccination, as immune therapy, dates back to the discovery by Jenner of the method of inoculating persons with cowpox against smallpox. Based on a knowledge that milkers once infected with cowpox were said to be unlikely to suffer from smallpox, he conceived the idea of artificially inoculating cowpox to prevent smallpox, and he verified the efficacy. Later Pasteur prevented disease by inoculating attenuated pathogens, thus demonstrating artificial induction of acquired immunity. The prophylactic effect of preventive inoculation, i.e., vaccination, relies on induction of acquired immunity, in which secondary and later immune responses are temporarily, quantitatively, and qualitatively superior to primary immune responses to initial antigen exposure. As such, acquired immunity relies mainly on T cells having the capability of antigen specific recognition, and being capable of memorizing the same. This is also evident from the fact that the onset of the pathology of acquired immunodeficiency due to HIV infection correlates with T lymphocyte dysfunction. Therefore, effective memorization of antigen-specific T lymphocytes is important to the establishment of acquired immune response by vaccination.

Acquired immune response is roughly divided into cellular immunity and humoral immune responses. To date acquired immune response by vaccination has been focused mainly on antibody production, a form of humoral immune response. In recent years, the importance of cellular immune response by vaccination has been emphasized, and the necessity of cytotoxic T lymphocytes (CTL), which are CD8-positive T lymphocytes being the major cells therein, was advocated. Currently, CTL induction by vaccination has not yet been optimized, and future technical innovations are awaited.

Conventional vaccines have been based mainly on viruses, microorganisms, components thereof and the like. From the viewpoint of developing safe and more effective vaccines, there is a demand for a vaccine comprising the minimum unit of active component. The minimum unit for antigen recognition by T cells is determined by a peptide presented by MHC molecules. CD8-positive T lymphocytes mainly recognize peptides of 8 to 11 residues presented by MHC class I molecules. If such a peptide in the minimum unit is used as a vaccine to induce effective CTL, a safer and more effective vaccine will be created.

Regarding this background, effective induction of memory CTL using a peptide in the minimum unit for specific recognition is important to the establishment of acquired immunity by a safer and more effective vaccine. The therapeutic effect of a peptide vaccine that has been clinically applied to date for cancers is low at about 5% of efficacy rate (see non-patent document 1). The specific CTL induction rates for peptide vaccines are low at an average of about 0.1 to 1% of CD8-positive T lymphocytes in the blood (see non-patent documents 2, 3 and 4). Meanwhile, the ratio of viral antigen specific CTL in CD8-positive cells in the blood exceeds 10% during acute viral infection (see non-patent document 5). Therefore, a peptide vaccine that could exhibit a CTL induction rate comparable with viral infection, if developed, will dramatically increase the cancer therapeutic effect.

To proliferate CTL to the extent found in acute viral infection, continuous amplification of CTL by repeated administration of a vaccine is important. For continuous proliferation of CTL, effective induction of memory CTL is important. Inductiion of memory CTL requires the help of CD4-positive T lymphocytes (see non-patent document 6).
Without the help of normal CD4-positive T lymphocytes, CTL will proliferate transiently upon antigen stimulation, but activation-dependent cell death will be induced and memory CTL necessary for the continuous amplification is not induced (see non-patent document 7). In the case of a vaccine consisting of peptide being the minimum unit for antigen recognition, help of normal CD4-positive T lymphocytes is unexpectable. Therefore, to induce continuously amplifiable memory CTL, it is necessary to add another factor to the peptide vaccine.

As factors involved in memory CTL induction comparable with the normal CD4 help, factors involved in (1) activation of antigen-presenting cells, (2) homeostatic proliferation after induction of lymphopenia, (3) suppression of regulatory T cells, and the like are known; although these factors have been reported to each exhibit an adjuvant action (CTL induction enhancement action), there is no report that CTL, in particular, continuously amplifiable memory CTL, was efficiently induced. Also, although it was reported the use of a CpG-DNA of a TLR9 ligand, which belongs to the aforementioned category (1), and an anti-CD25 antibody, which belongs to the aforementioned category (3), as a combination adjuvant, there was no clear additive or synergistic effect of the combination of both adjuvants on memory CTL induction by a peptide vaccine (recall response) (see non-patent document 8).

Non-patent document 1: Rosenberg SA et al., Nat. Med. 10: 909-915 (2004)
Non-patent document 2: Slingluff CL et al., Clin. Cancer Res. 7: 3012-3024 (2001)
Non-patent document 3: Schaed S et al., Clin. Cancer Res. 8: 967-972 (2002)
Non-patent document 4: Walker EB et al., Clin. Cancer Res. 10: 668-680 (2004)
Non-patent document 5: Hislop AD et al., J. Exp. Med. 197: 893-905 (2002)
Non-patent document 6: Janssen EM et al., Nature 421: 852-856 (2003)
Non-patent document 7: Janssen EM et al., Nature 434: 88-93 (2005)
Non-patent document 8: Toka FN et al., J.Viol. 78: 13082-13089 (2004)

### Disclosure of the Invention

### Problems to Be Solved by the Invention

It is an object of the present invention to provide a memory CTL induction enhancer comprising a combination of substance (b) with substance (a) and/or substance (c), wherein substance (a) is an activator of antigen-presenting cells, substance (b) is an inducer of homeostatic proliferation after induction of lymphopenia, and substance (c) is a suppressor of regulatory T cells.

### Means for Solving the Problems

The present inventor diligently investigated an adjuvant capable of effectively enhancing memory CTL induction by a vaccine (memory CTL induction enhancer). As a result, it was found possible to enhance memory CTL induction much more efficiently by means of:
- a combination of a substance belonging to (a) and a substance belonging to (b),
- a combination of a substance belonging to (b) and a substance belonging to (c), and
- a combination of a substance belonging to (a), a substance belonging to (b) and a substance belonging to (c),
wherein (a), (b) and (c) represent the categories of:
(a) activators of antigen-presenting cells,
(b) inducers of homeostatic proliferation after induction of lymphopenia, and
(c) suppressors of regulatory T cells, than the use of each substance alone, or the use of a conventionally known combination (a combination of a substance belonging to (a) and a substance belonging to (c)). In particular, it was found that memory CTL induction by a vaccine was dramatically improved by the aforementioned combination of 3 kinds of substances, and that continuous amplification of CTL by repeated administration of a vaccine was enabled by a combination of these three kinds of substances.
Accordingly, the present invention also provides the idea that in enhancing memory CTL induction, it is important to use a substance belonging to (b) above always in combination with a substance belonging to (a) above and/or a substance belonging to (c) above.

Specifically, the present inventor selected and investigated a TLR7 ligand (R848) (Hemmi H et al., Nat. Immunol. 3: 196-200 (2002)), a TLR9 ligand (CpG) (Hemmi H et al., Nature 408: 740-745 (2000)), and a TLR3 ligand (poly(A:U)) (Alexopoulou L et al., Nature 413: 732-738 (2001)), which are activators of toll-like receptors (TLR), as substances belonging to (a) above, cyclophosphamide (Lutsiak ME et al., 2005 Blood 105: 2862-2868) and carmustine (Maze R et al., J. Immunol. 158: 1006-1013 (1997)) as substances belonging to (b) above, and anti-CD25 antibody (Pasare C & Medzhitov R, 2003 Science 299: 1033-1036), anti-CD152 antibody (Takahashi T et al., 2000 J. Exp. Med. 192: 303-310), and anti-GITR antibody (Shimizu J et al., 2002 Nat. Immunol. 3: 135-142) as substances belonging to (c) above, and reached the above-described conclusion.

While cyclophosphamide had traditionally been viewed as a substance belonging to (c) above (Ercolini AM et al., J. Exp. Med. 201: 1591-1602 (2005)), a synergistic effect was observed when cyclophosphamide was used in combination with anti-CD25 antibody, a substance belonging to (c) above in Examples below; it was thus shown that cyclophosphamide belongs to (b) above, rather than to category (c). Because the present invention showed that cyclophosphamide was a substance belonging to (b) above, the idea of combining cyclophosphamide in combination with a substance belonging to (c) above being another category became possible.
The present invention has been developed on the basis of these findings.

Accordingly, the present invention relates to:
(1) a memory CTL induction enhancer comprising a combination of substance (b) with substance (a) and/or substance (c),
wherein substances (a), (b) and (c) are:
(a) an activator of antigen-presenting cells,
(b) an inducer of homeostatic proliferation after induction of lymphopenia, and
(c) a suppressor of regulatory T cells,

(2) the memory CTL induction enhancer of (1) above, comprising a combination of the aforementioned substance (a) and the aforementioned substance (b),
(3) the memory CTL induction enhancer of (2) above, wherein the aforementioned substance (a) and the aforementioned substance (b) are administered simultaneously, separately, or sequentially,
(4) the memory CTL induction enhancer of (2) above, wherein the memory CTL induction enhancer is a combination drug,
(5) the memory CTL induction enhancer of (2) above, wherein the memory CTL induction enhancer is a kit comprising a drug containing the aforementioned substance (a) and a drug containing the aforementioned substance (b),
(6) a memory CTL induction enhancer containing the aforementioned substance (b), to be used in combination with a drug containing the aforementioned substance (a),
(7) a memory CTL induction enhancer containing the aforementioned substance (a), to be used in combination with a drug containing the aforementioned substance (b),
(8) the memory CTL induction enhancer of any one of (2) to (7) above, wherein the aforementioned substance (a) is a TLR activator,
(9) the memory CTL induction enhancer of (8) above, wherein the TLR activator is a TLR7 ligand, a TLR9 ligand, or a TLR3 ligand,
(10) the memory CTL induction enhancer of any one of (2) to (9) above, wherein the aforementioned substance (b) is a cancer chemotherapeutic substance that induces lymphopenia,
(11) the memory CTL induction enhancer of (10) above, wherein the cancer chemotherapeutic substance that induces lymphopenia is cyclophosphamide or carmustine,
(12) the memory CTL induction enhancer of any one of (2) to (11) above, which is used along with an antigen protein or an antigen peptide derived from the antigen protein,
(13) the memory CTL induction enhancer of any one of (2) to (12) above, to be used to treat or prevent cancer or viral infectious disease,

(14) a method of enhancing the induction of memory CTL, comprising administering effective amounts of the aforementioned substance (a) and the aforementioned substance (b) as active components to a mammal,
(15) the method of enhancing the induction of memory CTL of (14) above, wherein the aforementioned substance (a) and the aforementioned substance (b) are administered simultaneously, separately, or sequentially,
(16) the method of enhancing the induction of memory CTL of (14) or (15) above, to be administered along with an antigen protein or an antigen peptide derived from the antigen protein,
(17) the method of enhancing the induction of memory CTL of any one of (14) to (16) above, for the treatment or prophylaxis of cancer or viral infectious disease,
(18) a use of the aforementioned substance (a) and the aforementioned substance (b) in the production of a memory CTL induction enhancer,
(19) the use of (18) above, to be used simultaneously, separately, or sequentially in the enhancement of memory CTL induction,
(20) the use of (18) or (19) above, to be used along with an antigen protein or an antigen peptide derived from the antigen protein in the enhancement of memory CTL induction,
(21) the use of any one of (18) to (20) above, to be used to treat or prevent cancer or viral infectious disease,

(22) the memory CTL induction enhancer of (1) above, comprising a combination of the aforementioned substance (b) and the aforementioned substance (c),
(23) the memory CTL induction enhancer of (22) above, wherein the aforementioned substance (b) and the aforementioned substance (c) are administered simultaneously, separately, or sequentially,
(24) the memory CTL induction enhancer of (22) above, wherein the memory CTL induction enhancer is a combination drug,
(25) the memory CTL induction enhancer of (22) above, wherein the memory CTL induction enhancer is a kit comprising a drug containing the aforementioned substance (b) and a drug containing the aforementioned substance (c),
(26) a memory CTL induction enhancer containing the aforementioned substance (c), to be used in combination with a drug containing the aforementioned substance (b),
(27) a memory CTL induction enhancer containing the aforementioned substance (b), to be used in combination with a drug containing the aforementioned substance (c),
(28) the memory CTL induction enhancer of any one of (22) to (27) above, wherein the aforementioned substance (b) is a cancer chemotherapeutic substance that induces lymphopenia, (29) the memory CTL induction enhancer of (28) above, wherein the cancer chemotherapeutic substance that induces lymphopenia is cyclophosphamide or carmustine,
(30) the memory CTL induction enhancer of any one of (22) to (29) above, wherein the aforementioned substance (c) is anti-CD4 antibody, anti-CD25 antibody, anti-CD152 antibody or anti-GITR antibody,
(31) the memory CTL induction enhancer of (30) above, wherein the aforementioned substance (c) is anti-CD25 antibody,
(32) the memory CTL induction enhancer of any one of (22) to (31) above, to be used along with an antigen protein or an antigen peptide derived from the antigen protein,
(33) the memory CTL induction enhancer of any one of (22) to (32) above, to be used to treat or prevent cancer or viral infectious disease,

(34) a method of enhancing the induction of memory CTL, comprising administering effective amounts of the aforementioned substance (b) and the aforementioned substance (c) as active components to a mammal,
(35) the method of enhancing the induction of memory CTL of (34) above, wherein the aforementioned substance (b) and the aforementioned substance (c) are administered simultaneously, separately, or sequentially,
(36) the method of enhancing the induction of memory CTL of (34) or (35) above, to be administered along with an antigen protein or an antigen peptide derived from the antigen protein,
(37) the method of enhancing the induction of memory CTL of any one of (34) to (36) above, for the treatment or prophylaxis of cancer or viral infectious disease,
(38) a use of the aforementioned substance (b) and the aforementioned substance (c) in the production of a memory CTL induction enhancer,
(39) the use of (38) above, to be used simultaneously, separately, or sequentially in the enhancement of memory CTL induction,
(40) the use of (38) or (39) above, to be used along with an antigen protein or an antigen peptide derived from the antigen protein in the enhancement of memory CTL induction,
(41) the use of any one of (38) to (40) above, to be used to treat or prevent cancer or viral infectious disease,

(42) the memory CTL induction enhancer of (1) above, comprising a combination of the aforementioned substance (a), the aforementioned substance (b) and the aforementioned substance (c),
(43) the memory CTL induction enhancer of (42) above, wherein the aforementioned substance (a), the aforementioned substance (b) and the aforementioned substance (c) are administered simultaneously, separately, or sequentially,
(44) the memory CTL induction enhancer of (42) above, wherein the memory CTL induction enhancer is a combination drug,
(45) the memory CTL induction enhancer of (42) above, wherein the memory CTL induction enhancer is a kit comprising a drug containing the aforementioned substance (a), a drug containing the aforementioned substance (b), and a drug containing the aforementioned substance (c),
(46) a memory CTL induction enhancer containing the aforementioned substance (c), to be used in combination with a drug containing the aforementioned substance (a) and a drug containing the aforementioned substance (b),
(47) a memory CTL induction enhancer containing the aforementioned substance (b), to be used in combination with a drug containing the aforementioned substance (a) and a drug containing the aforementioned substance (c),
(48) a memory CTL induction enhancer containing the aforementioned substance (a), to be used in combination with a drug containing the aforementioned substance (b) and a drug containing the aforementioned substance (c),
(49) a memory CTL induction enhancer containing the aforementioned substance (a), to be administered after administration of the aforementioned substance (b) and the aforementioned substance (c),
(50) a memory CTL induction enhancer containing the aforementioned substance (b) and/or the aforementioned substance (c), to be administered before administration of the aforementioned substance (a),
(51) the memory CTL induction enhancer of any one of (42) to (50) above, wherein the aforementioned substance (a) is a TLR activator,
(52) the memory CTL induction enhancer of (51) above, wherein the TLR activator is a TLR7 ligand, a TLR9 ligand, or a TLR3 ligand,
(53) the memory CTL induction enhancer of any one of (42) to (52) above, wherein the aforementioned substance (b) is a cancer chemotherapeutic substance that induces lymphopenia,
(54) the memory CTL induction enhancer of (53) above, wherein the cancer chemotherapeutic substance that induces lymphopenia is cyclophosphamide or carmustine,
(55) the memory CTL induction enhancer of any one of (42) to (54) above, wherein the aforementioned substance (c) is anti-CD4 antibody, anti-CD25 antibody, anti-CD152 antibody or anti-GITR antibody,
(56) the memory CTL induction enhancer of (55) above, wherein the aforementioned substance (c) is anti-CD25 antibody,
(57) the memory CTL induction enhancer of any one of (42) to (56) above, to be used along with an antigen protein or an antigen peptide derived from the antigen protein,
(58) the memory CTL induction enhancer of any one of (42) to (57) above, to be used to treat or prevent cancer or viral infectious disease,

(59) A method of enhancing the induction of memory CTL, comprising administering effective amounts of the aforementioned substance (a), the aforementioned substance (b) and the aforementioned substance (c) as active components to a mammal,
(60) the method of enhancing the induction of memory CTL of (59) above, wherein the aforementioned substance (a), the aforementioned substance (b) and the aforementioned substance (c) are administered simultaneously, separately, or sequentially,
(61) the method of enhancing the induction of memory CTL of (60) above, wherein the aforementioned substance (a) is administered after administration of the aforementioned substance (b) and the aforementioned substance (c),
(62) the method of enhancing the induction of memory CTL of any one of (59) to (61) above, administered along with an antigen protein or an antigen peptide derived from the antigen protein,
(63) the method of enhancing the induction of memory CTL of any one of (59) to (62) above, for the treatment or prophylaxis of cancer or viral infectious disease,
(64) a use of the aforementioned substance (a), the aforementioned substance (b) and the aforementioned substance (c) in the production of a memory CTL induction enhancer,
(65) the use of (64) above, to be used simultaneously, separately, or sequentially in the enhancement of memory CTL induction,
(66) the use of (65) above, wherein the aforementioned substance (a) is administered after administration of the aforementioned substance (b) and the aforementioned substance (c) in the enhancement of memory CTL induction,
(67) the use of any one of (64) to (66) above, to be used along with an antigen protein or an antigen peptide derived from the antigen protein in the enhancement of memory CTL induction,
(68) the use of any one of (64) to (67) above, to be used to treat or prevent cancer or viral infectious disease,

(69) an enhancer of memory CTL induction enhancement by (a) an activator of antigen-presenting cells and/or (c) a suppressor of regulatory T cells, containing (b) an inducer of homeostatic proliferation after induction of lymphopenia as an active component,
(70) an enhancer of memory CTL induction enhancement by (b) an inducer of homeostatic proliferation after induction of lymphopenia and/or (c) a suppressor of regulatory T cells, containing (a) an activator of antigen-presenting cells as an active component, and
(71) an enhancer of memory CTL induction enhancement by (a) an activator of antigen-presenting cells and/or (b) an inducer of homeostatic proliferation after induction of lymphopenia, containing (c) a suppressor of regulatory T cells as an active component.

### Effect of the Invention

The present invention is intended to efficiently enhance the induction of memory CTL by using substance (b) in combination with substance (a) and/or substance (c), wherein substance (a) is an activator of antigen-presenting cells, substance (b) is an inducer of homeostatic proliferation after induction of lymphopenia, and substance (c) is a suppressor of regulatory T cells. The memory CTL induction enhancer and method of induction enhancement of the present invention are effectively used in immune therapies for cancer or viral infectious disease.

### Best Mode for Carrying out the Invention

The present invention provides a memory CTL induction enhancer or method of induction enhancement comprising a combination of substance (b) with substance (a) and/or substance (c), wherein substances (a), (b) and (c) are:
(a) an activator of antigen-presenting cells,
(b) an inducer of homeostatic proliferation after induction of lymphopenia, and
(c) a suppressor of regulatory T cells,
(hereinafter, the memory CTL induction enhancer and method of induction enhancement are together also simply referred to as a memory CTL induction enhancer).

More specifically, the present invention provides a memory CTL induction enhancer or method of induction enhancement in the following three modes:
(1) a memory CTL induction enhancer or method of induction enhancement comprising a combination of the aforementioned substance (a) and the aforementioned substance (b),
(2) a memory CTL induction enhancer or method of induction enhancement comprising a combination of the aforementioned substance (b) and the aforementioned substance (c), and
(3) a memory CTL induction enhancer or method of induction enhancement comprising a combination of the aforementioned substance (a), the aforementioned substance (b) and the aforementioned substance (c).
The most preferable of them is the memory CTL induction enhancer or method of induction enhancement (3) comprising a combination of the aforementioned substance (a), the aforementioned substance (b) and the aforementioned substance (c).

The above-mentioned "(a) an activator of antigen-presenting cells" is not particularly limited, as long as it is a substance conventionally known as having antigen-presenting cell activating action. Representative examples include activators of Toll-like receptors (TLR) (Akira S & Hemmi H Immunol. Lett. 85: 85-95 (2003)).

The activators of TLR specifically include TLR2 ligands, TLR2/4 dual ligands, TLR3 ligands, TLR4 ligands, TLR5 ligands, TLR7 ligands, TLR9 ligands and the like.

TLR2 ligands include, for example, peptideglycans, lipoproteins, glycolipids, lipoarabinomannan, Zymosan, outer membrane protein A and the like.
TLR2/4 dual ligands include, for example, LPS, Lipid A, Heat Shock Protein and the like.
TLR3 ligands include, for example, double-stranded RNA, poly(I:C), poly(A:U) and the like.
TLR4 ligands include, for example, Flavolipin, F protein, Taxol and the like.
TLR5 ligands include, for example, Flagellin and the like.
TLR7 ligands include, for example, imidazoquinoline compounds (R837, R848), adenine compounds, single-stranded RNA, Loxoribine and structural analogues thereof, Bropirimine and structural analogues thereof and the like.
TLR9 ligands include, for example, immunostimulatory DNA containing the CpG or CpR sequence and the like.
Of these substances, TLR7 ligands, TLR9 ligands, and TLR3 ligands are preferable; TLR7 ligands are more preferable.

All the substances mentioned above are commonly known substances. Structures and methods of production of adenine compounds out of them are described in WO 98/1448, WO 99/28321, WO 02/85905 and the like. Structures and methods of production of single-stranded RNA are described in WO 03/086280, Heil F et al., Science 303: 1526-1529 (2004), Diebold SS et al., Science 303: 1529-1531 (2004) and the like. Structures and methods of production of CpR immunostimulatory DNA are described in Kandimalla ER et al., Proc. Natl. Acad. Sci. USA 102: 6925-6930 (2005) and the like. Structures and methods of production of the other substances mentioned above are described in Akira S & Hemmi H Immunol. Lett. 85: 85-95 (2003) and the like.
The fact that TLR activation has adjuvant activity is shown in, for example, Pasare C & Medzhitov R, Immunity 21: 733-741 (2004) and the like.

The above-mentioned "(b) an inducer of homeostatic proliferation after induction of lymphopenia" is not particularly limited, as long as it is a substance conventionally known as having an action to induce homeostatic proliferation after induction of lymphopenia, whether it mediates induction of lymphopenia or induction of homeostatic proliferation. Representative examples include cancer chemotherapeutic substances that induce lymphopenia (cancer chemotherapeutic agents), radiation (irradiation), T cell growth factors whose receptors are common γ chains that induce homeostatic proliferation, and the like.

Cancer chemotherapeutic substances that induce lymphopenia include, for example, cyclophosphamide or carmustine (Morrissey PJ et al., J. Immunol. 146: 1547-1552 (1991) and Maze R et al., J. Immunol. 158: 1006-1013 (1997)).
Examples of radiations irradiated to induce lymphopenia include X rays, γ rays, electron beams, proton beams, heavy-particle beams and the like (Peacock CD et al., J. Immunol. 171: 655-663 (2003)).

T cell growth factors whose receptors are common γ chains that induce homeostatic proliferation include, for example, IL-2, IL-4, IL-7, IL-9, IL-15, IL-21 and the like (Kovanen PE & Leonard WJ, Immunol. Rev. 202: 67-83 (2004)).
Of these substances, cancer chemotherapeutic substances that induce lymphopenia are preferable. The cancer chemotherapeutic substances are preferably cyclophosphamide and carmustine; cyclophosphamide is more preferable.

All the substances mentioned above are commonly known; for the structures and methods of production thereof, see the aforementioned references and the like.
Representatively, the fact that cyclophosphamide has adjuvant activity is shown in, for example, Ghiringhelli F et al., Eur. J. Immunol. 34: 336-344 (2004), Lutsiak ME et al., Blood 105: 2862-2868 (2005), Bosco N et al., J. Immunol. 175: 162-170 (2005), Mihalyo MA et al., J. Immunol. 172: 5338-5345 (2004) and the like.

The above-mentioned "(c) a suppressor of regulatory T cells" is not particularly limited, as long as it is a substance conventionally known as having suppressive action on regulatory T cells. Representative examples include antibodies that bind to surface antigens on regulatory T cells and synthetic low-molecular compounds.
Antibodies that bind to surface antigens on regulatory T cells include, for example, anti-CD4 antibody, anti-CD25 antibody, anti-CD152 antibody, anti-GITR antibody and the like (Takahashi T et al., 2000 J. Exp. Med. 192: 303-310, Shimizu J et al., 2002 Nat. Immunol. 3: 135-142).

Low-molecular compounds that suppress regulatory T cells include, for example, P2X7R ligands, ATP, NAD, benzoylbenzoyl ATP and the like (Aswad F et al., 2005 J. Immunol. 175: 3075-3083).
Of these substances, antibodies that bind to surface antigens on regulatory T cells are preferable. Preferably, anti-CD4 antibody, anti-CD25 antibody, anti-CD152 antibody and anti-GITR antibody may be mentioned. Anti-CD25 antibody is more preferable.

All the substances mentioned above are commonly known; for the structures and methods of production thereof, see the aforementioned references.
Representatively, the fact that anti-CD25 antibody has adjuvant activity is shown in, for example, Casares N et al., J. Immunol. 171: 5931-5989 (2003), Toka FN et al., J. Virol. 78: 13082-13089 (2004), Yu P et al., J. Exp. Med. 201: 779-791 (2005) and the like.

The memory CTL induction enhancer of the present invention may be any combination, as long as it concerns:
(1) a combination of the aforementioned substance (a) and the aforementioned substance (b),
(2) a combination of the aforementioned substance (b) and the aforementioned substance (c), or
(3) a combination of the aforementioned substance (a), the aforementioned substance (b) and the aforementioned substance (c). Not only 1 kind, but also 2 or more kinds of substances can be selected from each of the categories (a), (b), and (c).

As the above-described combination (1), i.e., "a combination of the aforementioned substance (a) and the aforementioned substance (b)", a combination of (a) a TLR activator and (b) a cancer chemotherapeutic substance that induces lymphopenia is preferable. In particular, a combination of (a) a TLR7 ligand, a TLR9 ligand or a TLR3 ligand and (b) cyclophosphamide or carmustine is preferable.
As the above-described combination (2), i.e., "a combination of the aforementioned substance (b) and the aforementioned substance (c)", a combination of (b) a cancer chemotherapeutic substance that induces lymphopenia and (c) an antibody that binds to a surface antigen on regulatory T cells is preferable. In particular, a combination of (b) cyclophosphamide or carmustine and (c) anti-CD25 antibody, anti-CD152 antibody or anti-GITR antibody is preferable. Particularly, a combination of (b) cyclophosphamide and (c) anti-CD25 antibody is preferable.

As the above-described combination (3), i.e., "a combination of the aforementioned substance (a), the aforementioned substance (b) and the aforementioned substance (c)", a combination of (a) a TLR activator, (b) a cancer chemotherapeutic substance that induces lymphopenia, and (c) an antibody that binds to a surface antigen on regulatory T cells is preferable. In particular, a combination of (a) a TLR7 ligand, a TLR9 ligand or a TLR3 ligand, (b) cyclophosphamide or carmustine, and (c) anti-CD25 antibody, anti-CD152 antibody or anti-GITR antibody is preferable. Particularly, a combination of (a) a TLR7 ligand, (b) cyclophosphamide, and (c) anti-CD25 antibody is preferable.
Of the aforementioned combinations (1), (2), and (3), the combination (3), i.e., "a combination of the aforementioned substance (a), the aforementioned substance (b) and the aforementioned substance (c)" is particularly preferable.

The memory CTL induction enhancer of the present invention can be used along with an antigen protein or an antigen peptide (vaccine) derived from the antigen protein, so as to enhance the induction of memory CTL by the antigen protein or antigen peptide. On the other hand, it is also possible to enhance the induction of endogenous memory CTL being present in living organisms.

When the memory CTL induction enhancer of the present invention is used along with an antigen protein or an antigen peptide, the antigen protein and antigen peptide are not particularly limited, as long as they are a conventional commonly known antigen protein and antigen peptide. Specifically, such antigen proteins and antigen peptides include cancer antigen proteins and cancer antigen peptides derived therefrom, and viral antigen proteins and viral antigen peptides derived therefrom.

Cancer antigen proteins include, for example, MAGE (Science, 254: p1643 (1991)), gp100 (J. Exp. Med., 179: p1005 (1994)), MART-1 (Proc. Natl. Acad. Sci. USA, 91: p3515 (1994)), tyrosinase (J. Exp. Med., 178: p489 (1993)), MAGE-related proteins (J. Exp. Med., 179: p921 (1994)), β-catenin (J. Exp. Med., 183: p1185 (1996)), CDK4 (Science, 269: p1281 (1995)), HER2/neu (J. Exp. Med., 181: p2109 (1995)), mutant p53 (Proc. Natl. Acad. Sci. USA, 93: p14704 (1996)), CEA (J. Natl. Cancer. Inst., 87: p982 (1995)), PSA (J. Natl. Cancer. Inst., 89: p293 (1997)), WT1 (Proc. Natl. Acad. Sci. USA, 101: p13885 (2004)), HPV (J. Immunol., 154: p5934 (1995)), EBV (Int. Immunol., 7: p653 (1995)) and the like.

Viral antigen proteins include antigen proteins derived from viruses such as HIV, hepatitis C virus, hepatitis B virus, influenza virus, HPV, HTLV, and EBV.

The memory CTL induction enhancer of the present invention is capable of enhancing the induction of memory CTL at high efficiency that has not been achieved conventionally, and can therefore also be used in combination with an antigen peptide (peptide vaccine) that exhibits a low specific CTL induction rate. Some specific examples of antigen peptides are shown below (each numerical figure indicates a position on the amino acid sequence of the cancer antigen protein).

(1) Cancer antigen peptides derived from cancer antigens
•MAGEA3 peptide 168-176 (Coulie PG et al., Immunol. Rev. 188: 33 (2002)),
•gp100 peptide 209-217 (Rosenberg SA et al., Nat. Med. 4: 321 (1998)),
•Melan-A peptide 27-35 (Cormier JN et al., Cancer J. Sci. Am. 3: 37 (1997)),
•Melan-A peptide 26-35, Tyrosinase peptide 1-9, Tyrosinase peptide 368-376, gp100 peptide 280-288, gp100 peptide 457-466 (Jager E et al., Int. J. Cancer 67: 54 (1996)),
•HER-2 peptide 369-384, HER-2 peptide 688-703, HER-2 peptide 971-984 (Knutson KL et al., J. Clin. Invest. 107: 477 (2001)),
•MAGE-A12 peptide 170-178 (Bettinotti MP et al., Int. J. Cancer 105: 210 (2003)),
•gp100 peptide 280-288 (Phan GQ et al., Proc. Natl. Acad. Sci. USA 100: 8372 (2003))

(2) Viral antigen peptides derived from viral antigens
•Influenza matrix protein peptide 58-66 (Jager E et al., Int. J. Cancer 67: 54 (1996)),
•HPV16 E7 peptide 86-93 (van Driel WJ et al., Eur. J. Cancer 35: 946 (1999)),
•HPV E7 peptide 12-20 (Scheibenbogen C et al., J. Immunother 23: 275 (2000)),
•HPV16 E7 peptide 11-20 (Smith JWI et al., J. Clin. Oncol. 21: 1562 (2003))

An antigen protein can be prepared by cloning a cDNA that encodes the antigen protein, and expressing the same in host cells according to textbooks such as Molecular Cloning 2nd Edt., Cold Spring Harbor Laboratory Press (1989).
Synthesis of an antigen peptide can be performed in accordance with a method in use for ordinary peptide chemistry. Such methods of synthesis include methods described in references (Peptide Synthesis, Interscience, New York, 1966; The Proteins, Vol. 2, Academic Press Inc., New York, 1976; Peptide Gosei, Maruzen Co., 1975; Peptide Gosei-no-Kiso to Jikken (Basics and experiments of peptide synthesis), Maruzen Co., 1985; Zoku Iyakuhin no Kaihatsu (A Sequel to Development of Pharmaceuticals), Vol. 14, Peptide Synthesis, Hirokawa Shoten, 1991) and the like.

The memory CTL induction enhancer of the present invention is used for the treatment or prophylaxis of cancer or viral infectious disease.

Being active components of the memory CTL induction enhancer of the present invention, each of the substances belonging to the categories (a), (b), and (c):
(a) activators of antigen-presenting cells,
(b) inducers of homeostatic proliferation after induction of lymphopenia, and
(c) suppressors of regulatory T cells,
can be mixed with a physiologically acceptable carrier, excipient, binder, diluent, emulsifier and the like to obtain preparations. The amount of each substance in the preparation is not particularly limited, as long as memory CTL induction enhancement is possible. For example, a parenteral preparation may be prepared so that the dosage of each substance is normally 0.0001 mg to 1000 mg, preferably 0.001 mg to 100 mg, more preferably 0.01 mg to 10 mg, per dose.

Being active components of the memory CTL induction enhancer of the present invention, the aforementioned substances (a), (b), and (c) can be administered parenterally or orally when used as pharmaceutical preparations. Specifically, the substances (a), (b), and (c) can be administered as injectable preparations prepared in the form of liquid preparations such as solutions, emulsions, and suspensions, and, as required, a buffering agent, a solubilizer, and an isotonizing agent and the like can be added. The substances (a), (b), and (c) can also be administered rectally in the form of suppositories. The substances (a), (b), and (c) can also be administered orally in dosage forms for ordinary use, for example, tablets, capsules, syrups, suspensions and the like. Such dosage forms can be produced by blending an ordinary carrier, excipient, binder, stabilizer and the like and active components (substances of the present invention having memory CTL induction enhancement action), according to a common method.

Although the method of administering the memory CTL induction enhancer of the present invention may be any of oral administration and parenteral administration, parenteral administration is preferable. In the case of parenteral administration, subcutaneous injection, continuous subcutaneous injection, intradermal injection, intravenous injection, intraarterial injection, muscular injection; intraperitoneal administration, transdermal administration, transmucosal administration, nasal administration and the like may be mentioned. It is also possible to continuously and gradually administer the memory CTL induction enhancer of the present invention using an osmotic pump and the like, and to prepare and embed a sustained-release preparation. The individual components of the memory CTL induction enhancer of the present invention (the aforementioned substances (a), (b), and (c)) may be administered by the same method of administration (route of administration) or by different methods of administration (routes of administration). Frequency of administration is not particularly limited; usually, administration is performed once per several days to several months, or consecutive-day administration is performed with a washout of several days to several months.

As long as the memory CTL induction enhancer of the present invention concerns:
(1) a combination of the aforementioned substance (a) and the aforementioned substance (b),
(2) a combination of the aforementioned substance (b) and the aforementioned substance (c), or
(3) a combination of the aforementioned substance (a), the aforementioned substance (b) and the aforementioned substance (c),
it can be used in any of the forms:
(A) A form of a combination drug (mixture) obtained by mixing substances concerning the aforementioned combinations.
(B) A form of a kit comprising drugs each containing individual substance concerning the aforementioned combinations.
(C) A combination form wherein drugs each containing individual substance concerning the aforementioned combinations are administered simultaneously, separately, or sequentially.
Of these forms, (A), (B) and (C) concerning the aforementioned combination (3) are preferable.

When the memory CTL induction enhancer of the present invention is a combination drug (mixture), the blending ratio of the aforementioned substance (a) and the aforementioned substance (b), the blending ratio of the aforementioned substance (b) and the aforementioned substance (c), and the blending ratio of the aforementioned substances (a), (b) and (c) are not particularly limited. The blending ratio may be such that the individual active components are contained in the memory CTL induction enhancer so that they are administered at the above-described dosages, per dose of the memory CTL induction enhancer. The combination drug may be a preparation prepared as a combination drug, and may be prepared (mixed) freshly before use for administration to a patient.

When the memory CTL induction enhancer of the present invention is a kit, the aforementioned substance (a), the aforementioned substance (b), and the aforementioned substance (c) are separately prepared and enclosed in separate containers. A kit is a form wherein containers each containing one of these drugs are enclosed in a single packaging container.
The individual drugs (individual components) contained in a kit can be used after being freshly prepared (mixed) before use for administration to a patient. The individual drugs (individual components) contained in the kit can also be used simultaneously, separately, or sequentially (for details see the forms of combination shown below).

When the memory CTL induction enhancer of the present invention is administered simultaneously, separately, or sequentially in combination form, the aforementioned substance (a), the aforementioned substance (b), and the aforementioned substance (c) are prepared separately and enclosed in separate containers. The methods of administration (routes of administration such as subcutaneous injection, intradermal injection, and intravenous injection) of the aforementioned substance (a), the aforementioned substance (b), and the aforementioned substance (c) administered simultaneously, separately, or sequentially may be the same methods of administration, or different methods of administration.
In the case of separate administration, the aforementioned substance (a), the aforementioned substance (b), and the aforementioned substance (c) may be administered in any order, and frequency of administration of each substance is not limited, as long as memory CTL induction enhancement action is observed.
In the case of sequential administration, the aforementioned substance (a), the aforementioned substance (b), and the aforementioned substance (c) may be administered in any order, and frequency of administration of each substance is not limited, as long as memory CTL induction enhancement action is observed.

In the case of separate or sequential administration, the aforementioned substance (a), the aforementioned substance (b), and the aforementioned substance (c) may be administered at any intervals, whether one substance is administered immediately after administration of the foregoing substance, or after an interval of about 1 day to 6 months.
Administration after an interval of 1 day to 2 weeks is preferable.
Preferable methods of administration are described below.

Because the individual substances having the actions of activation of antigen-presenting cells, induction of homeostatic proliferation after induction of lymphopenia, and suppression of regulatory T cells, respectively (the aforementioned substances (a), (b), and (c)) have different times for the manifestation of the effects thereof, it is desirable, to maximize the effects, that they should be administered in combination with a vaccine in consideration of the optimum times for the manifestation of the respective effects.

For example, it is known that R848, a TLR7 ligand that induces the activation of antigen-presenting cells, exhibits cytokine production from 1 hour after administration (Hemmi H et al., 2002 Nat. Immunol. 3: 196-200). It is also known that when anti-CD25 antibody, which induces the suppression of regulatory T cells, is administered, the amount of regulatory T cells is minimized 3 days after administration (Pasare C & Medzhitov R, 2003 Science 299: 1033-1036). Furthermore, it is known that when cyclophosphamide, which induces homeostatic proliferation after induction of lymphopenia, is administered, the reduction in lymphocytes reaches a peak after 3 to 4 days (Lutsiak ME et al., 2005 Blood 105: 2862-2868).

Therefore, for example, it is thought that the resulting effect is maximized by administering the aforementioned substance (c), which mediates the suppression of regulatory T cells, and the aforementioned substance (b), which mediates homeostatic proliferation after induction of lymphopenia, on the same day, and, about 3 days later, administering the aforementioned substance (a), which induces the activation of antigen-presenting cells, along with a vaccine.

Therefore, preferable methods of administration according to the present invention are exemplified by the following methods of administration:
(1) In case where the aforementioned substance (a) and the aforementioned substance (b) are used in combination:
   The aforementioned substance (a) is administered after administration of the aforementioned substance (b).
(2) In case where the aforementioned substance (b) and the aforementioned substance (c) are used in combination:
   The aforementioned substance (b) and the aforementioned substance (a) are administered on the same day.
(3) In case where the aforementioned substance (a), the aforementioned substance (b) and the aforementioned substance (c) are used in combination:
   The aforementioned substance (a) is administered after administration of the aforementioned substance (b) and the aforementioned substance (c).
Of these methods, the method wherein the aforementioned substance (a) is administered after administration of the aforementioned substance (b) and the aforementioned substance (c) is particularly preferable.

The present invention is hereinafter described in more detail by means of the following Examples, by which, however, the present invention is never limited.

### Example 1

### Investigation of optimum combination of enhancers of memory CTL induction by vaccine

It is difficult to directly detect the proliferation of specific CTL by vaccine administration because of low content. Hence, the content was increased using a CTL clone (OT-I) that proliferates specifically in the presence of a model peptide (OVA257-264 peptide) used as a vaccine, and the actions of memory CTL induction enhancers were compared. Cyclophosphamide, anti-CD25 antibody, and the TLR activator R848 (TLR7 ligand) were investigated to enhance induction of memory CTL.

The spleen was excised from each OT-I mouse (C57BL/6-Tg (OT-I)-RAG1^{tmlMom}, Taconic) (Mombaerts P et al., 1992 Cell 68: 869-877, Hogquist KA et al., 1994 Cell 76: 17-27), and mashed by the frost portion of a glass slide. Erythrocytes were lysed with ACK buffer (0.15M NH₄Cl, 10mM KHCO₃, 0.1mM EDTA, pH7.2-7.4), the lysate was filtered through Nylon mesh, and splenocytes were prepared. 5 x 10⁵ OT-I mouse splenocytes suspended in PBS were intravenously administered to the tail vein of each wild mouse (C57BL/6NCrj, Charles River Japan Inc.) (300 µL/animal).
15 mg/ml cyclophosphamide (SIGMA) was prepared using PBS, and intraperitoneally administered on the day after transfer of OT-I mouse splenocytes (150 mg/kg, 10 ml/kg). For the control group, PBS was administered.

Furthermore, on the same day, anti-CD25 antibody (clone: PC61, BD Pharmingen) was intravenously administered to the tail vein at 50 µg/300 µl/mouse. For the control group, an isotype antibody (clone: A110-1, BD Pharmingen) was intravenously administered to the tail vein at 50 µg/300 µl/mouse.
100 µg of the OVA257-264 peptide alone (sequence: SIINFEKL), or both 100 µg of the OVA257-264 peptide and 50 µg of R848 (the method of synthesis is disclosed in WO 94/17043) dissolved in PBS, were subcutaneously administered at the base of the tail of each mouse (100 µl) 3 days after administration of cyclophosphamide and PC61 antibody.
Twenty-one days after initial peptide administration, 100 µg of the OVA257-264 peptide dissolved in PBS, alone, was again subcutaneously administered at the base of the tail of each mouse.

Four days after initial peptide administration, or 4 days after re-administration, the spleen was excised, and splenocytes were prepared using frost glass slides, ACK buffer, Nylon mesh and the like. The splenocytes were washed with 2% FCS/PBS, and sown to a 96-well V-bottomed plate at 3 x 10⁶ cells/100 µl. 2 µl of anti-mouse CD16/CD32 (clone: 2.4G2, BD Pharmingen) was added, and the cells were incubated at 4°C for 15 minutes to prevent non-specific adsorption due to blocking of the Fc site. 5 µl of PE conjugated H-2Kb tetramer /SIINFEKL (Immuno-Biological Laboratories Co., Ltd.) and 2 µl of PerCP conjugated anti-CD8a antibody (clone: 53-6.7, BD Pharmingen) were added, and the cells were incubated at room temperature for 30 minutes. The stained cells were washed with 2% FCS/PBS, and fixed with 1% formalin/PBS. The stained cells were analyzed using FACSCan (Becton Dickinson), and the specific tetramer positive rate in the CD8-positive cells was determined using the CELLQuest data analysis software (Becton Dickinson).

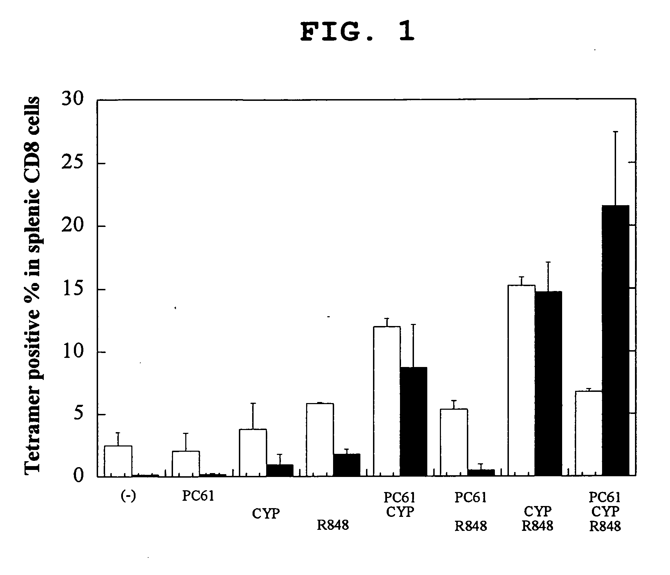

The ratios of the peptide specific tetramer positive CD8 T lymphocytes in the spleen after initial administration of the peptide with various enhancers and after re-administration of the peptide alone are shown in FIG. 1. It was found that memory CTL induction was efficiently enhanced by a combination of anti-CD25 antibody (PC61) and cyclophosphamide, a combination of cyclophosphamide and R848, and a combination of the three components of CD25 antibody (PC61), cyclophosphamide, and R848.

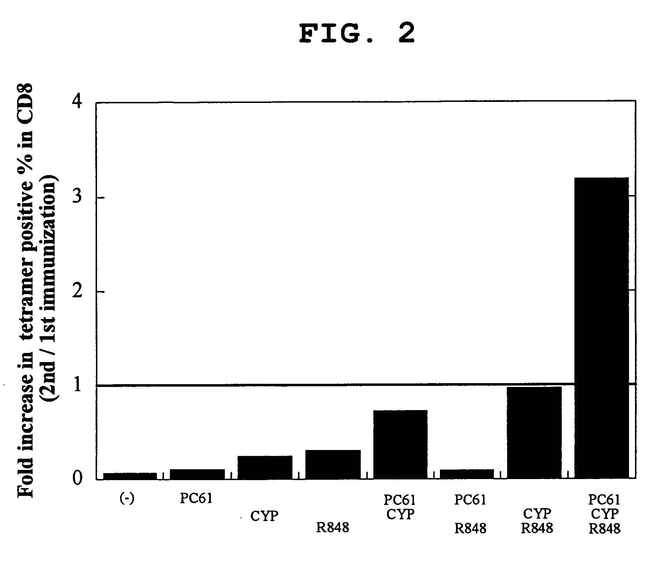

Furthermore, to determine the ratio of memory CTLs capable of proliferating after re-administration to the specific CTLs that proliferated after initial administration, the percentage of the tetramer positive rate after re-administration to the tetramer positive rate after initial administration was calculated. The results are shown in FIG. 2. With a combination of anti-CD25 antibody (PC61) and cyclophosphamide, and with a combination of cyclophosphamide and R848, compared with each drug alone, increased memory CT induction was observed. Furthermore, when this memory induction rate is 1 or more, continuous amplification of memory CTL induction by repeated administration is expected. As shown in FIG. 2, it was found that by combining 3 kinds of memory CTL induction enhancers with different mechanisms (cyclophosphamide, anti-CD25 antibody, TLR7 ligand R848), memory induction by a vaccine was dramatically improved.

### Example 2

### Continuous amplification of memory CTL with vaccine and a combination of 3 kinds of memory CTL induction enhancers

From Example 1, it was found that memory CTL induction by a vaccine was dramatically enhanced by combining 3 kinds of memory CTL induction enhancers (cyclophosphamide, anti-CD25 antibody, R848). Next, an investigation was performed to determine whether this combination of memory CTL induction enhancers enhances memory induction by the vaccine even without introduction of OT-I cells, and whether the memory induction is amplified continuously.

Cyclophosphamide, prepared in the same manner as Example 1, was intraperitoneally administered to each naive wild mouse (150 mg/kg, 10 ml/kg). Furthermore, on the same day, anti-CD25 antibody (clone: PC61), an antibody which depletes regulatory T cells, was intravenously administered to the tail vein at 50 µg/300 µl/mouse. Three days later, 100 µg of the OVA257-264 peptide and 50 µg of R848 dissolved in PBS were subcutaneously administered at the base of the tail of each mouse (100 µl). This was repeated at intervals of 2 weeks or 3 weeks.

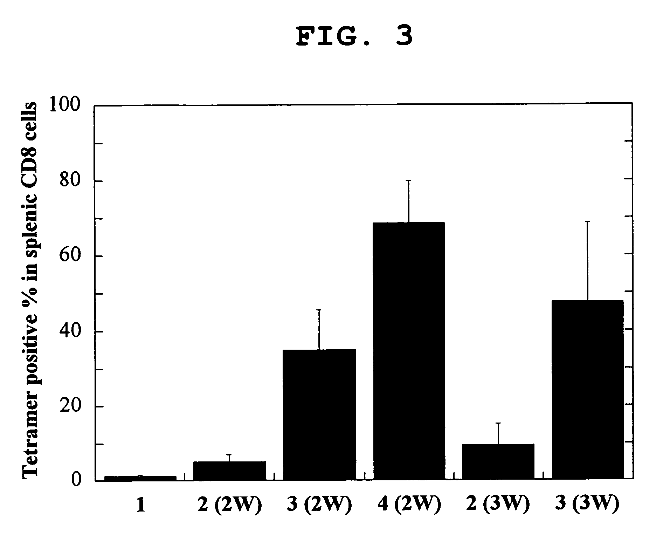

Seven days after final administration, the spleen was excised, and the ratio of OVA257-264 peptide specific H-2Kb tetramer-positive cells in CD8-positive cells was determined in the same manner as Example 1.
When the peptide vaccine was repeatedly administered in combination with the 3 kinds of memory CTL induction enhancers, it was found that tetramer-positive cells could be detected by repeating administration twice or more, and that specific CTLs proliferated depending on the number of administration (FIG. 3). From this result, it was found possible to induce continuously amplifiable memory CTL by adding a combination of the 3 kinds of memory CTL induction enhancers (cyclophosphamide, anti-CD25 antibody, R848) to the peptide vaccine.

### Example 3

### Comparison of continuous amplification of memory CTL by combination of 2 kinds or 3 kinds of memory CTL induction enhancers

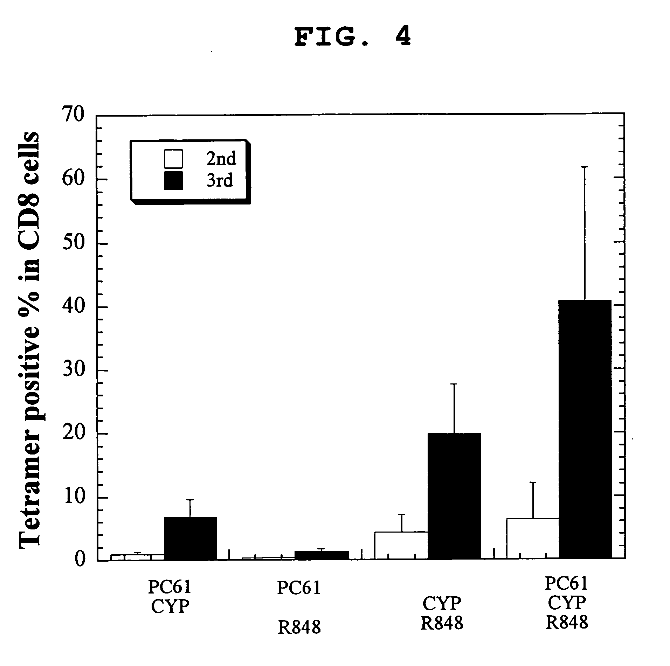

In Example 2, it was found that by combining 3 kinds of memory CTL induction enhancers (cyclophosphamide, anti-CD25 antibody, R848), specific CTLs proliferated depending on the number of administration even without OT-I cells. Next, without OT-I cells as in Example 2, specific CTL proliferation depending on the number of administration by combination of 2 or 3 kinds of memory CTL induction enhancers (cyclophosphamide, anti-CD25 antibody, R848) was compared. As a result, CTL induction action depending on the number of administration was detected when a combination of anti-CD25 antibody (PC61) and cyclophosphamide and a combination of cyclophosphamide and R848 were administered. A combination of the 3 kinds (cyclophosphamide, anti-CD25 antibody, R848) was more potent than the combinations of 2 kinds in terms of CTL induction action depending on the number of administration (FIG. 4).

### Example 4

### In vivo antitumor effect of memory CTL induced by combination of memory CTL induction enhancers

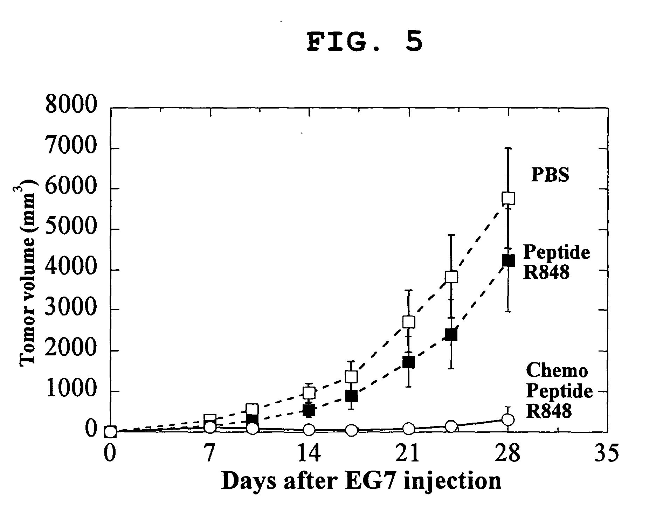

The tumor growth suppressing action in vivo of CTL induced by a peptide vaccine and a combination of 3 kinds of memory CTL induction enhancers (cyclophosphamide, anti-CD25 antibody, R848) was investigated. The peptide vaccine and the 3 kinds of memory CTL induction enhancers (cyclophosphamide, anti-CD25 antibody, R848) were administered to wild mice (C57BL/6NCrj, Charles River Japan Inc.) under the same conditions as Example 1. Twenty-one days after peptide administration, 5 x 10⁶ EG7 (OVA-expressing EL4: ATCC) suspended in 100 µL PBS was intradermally transplanted at the right flank using a 27G injection needle after shaving. Tumor diameters (minimum diameter, maximum diameter) were measured using electronic calipers twice a week from 1 week to 28 days after EG7 transplantation, and tumor volumes were calculated. As a result, a slight suppression against proliferation was observed with administration of the peptide vaccine and R848 in combination compared with PBS administration. In contrast, when the peptide vaccine and the 3 kinds of memory CTL induction enhancers (cyclophosphamide, anti-CD25 antibody, R848) were administered in combination, tumor growth was nearly completely suppressed until day 28 after tumor transplantation (FIG. 5). Hence, it was found that a combination of the peptide vaccine and the 3 kinds of memory CTL induction enhancers (cyclophosphamide, anti-CD25 antibody, R848) induced CTLs that potently suppresses tumor growth in vivo.

### Example 5

### Enhancement of memory CTL by combinations of other memory CTL induction enhancers

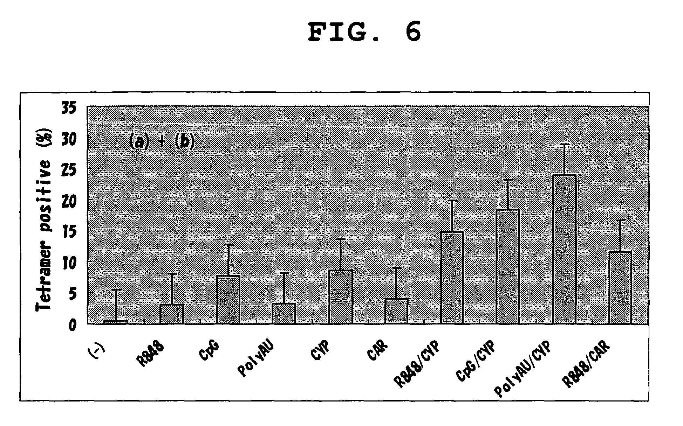

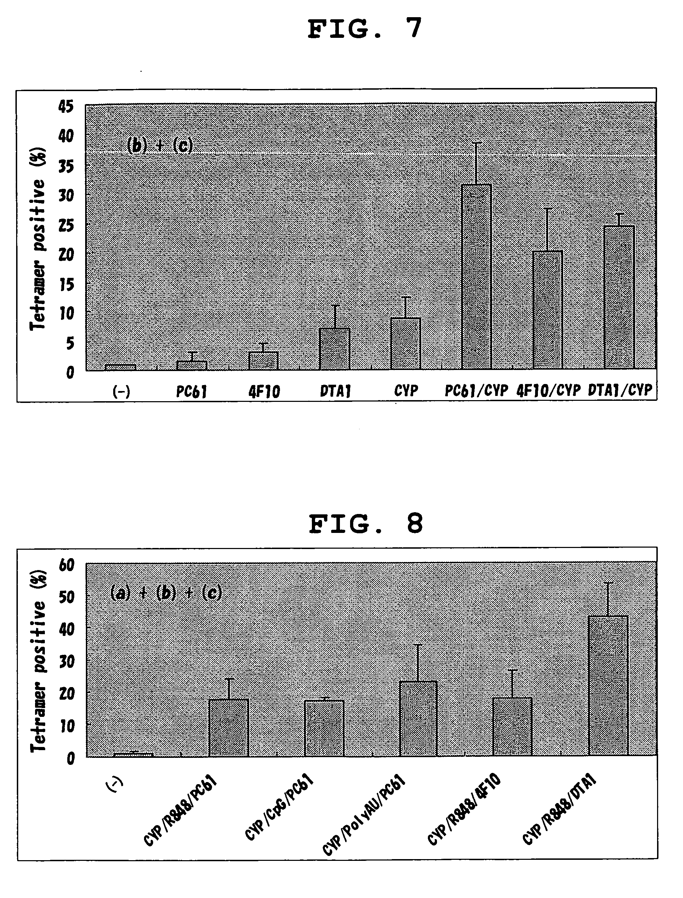

In the investigations described above, R848 from category (a) activators of antigen-presenting cells, cyclophosphamide from category (b) inducers of homeostatic proliferation after induction of lymphopenia, and anti-CD25 antibody from category (c) suppressors of regulatory T cells, were used. Furthermore, the effects of combinations of 2 kinds or 3 kinds of other substances were investigated. The combination effects of (a) + (b), (b) + (c), and (a) + (b) + (c) were investigated wherein (a) was CpG (Hokkaido System Science Co., Ltd.), a TLR9 ligand, or poly(A:U) (SIGMA), a TLR3 ligand, (b) was carmustine (SIGMA), and (c) was anti-CD152 antibody (clone 4F10: BD Pharmingen) or anti-GITR antibody (clone DTA1: eBioscience). The amount of peptide specific CTL in the spleen was quantified by using of a tetramer at 4 days after re-administration of the peptide alone using OT-I mice as in Example 1, under the same administration conditions. As a result, both in FIG. 6 ((a) + (b)) and FIG. 7 ((b) + (c)), a remarkable combination effect was observed compared with each drug alone. Referring to FIG. 8 ((a) + (b) + (c)), all combinations exhibited equivalent or more CTL induction capability compared with that of the already investigated combination of R848, cyclophosphamide, and anti-CD25 antibody (PC61). Hence, all the combinations of substances (a) + (b), (b) + (c), and (a) + (b) + (c) investigated here exhibited a combination effect for the enhancement of memory CTL induction.

### Industrial Applicability

Provided by the present invention is a memory CTL induction enhancer comprising a combination of substance (b) with substance (a) and/or substance (c), wherein substance (a) is an activator of antigen-presenting cells, substance (b) is an inducer of homeostatic proliferation after induction of lymphopenia, and substance (c) is a suppressor of regulatory T cells. The memory CTL induction enhancer of the present invention is capable of efficiently enhancing memory CTL induction, and can therefore be effectively used in immune therapies for cancer or viral infectious disease.

### Brief Description of the Drawings

[FIG. 1] A graph showing the tetramer positive rate in the spleen after first administration of a peptide vaccine with the addition of various memory CTL induction enhancers (anti-CD25 antibody: PC61, cyclophosphamide: CYP, TLR7 ligand: R848) and a combination thereof, or without the addition (indicated by (-)) (white bar), and the tetramer positive rate in the spleen after re-administration of the peptide alone (black bar).
[FIG. 2] A graph showing the memory CTL induction rate by a peptide vaccine with the addition of various memory CTL induction enhancers (anti-CD25 antibody: PC61, cyclophosphamide: CYP, TLR7 ligand: R848) and a combination thereof, or without the addition (indicated by (-)).
[FIG. 3] A graph showing the results of measurements of the tetramer positive rate in the spleen at 1 week after final administration in repeats of administration of 3 kinds of memory CTL induction enhancers (anti-CD25 antibody: PC61, cyclophosphamide: CYP, TLR7 ligand: R848) and a peptide vaccine at intervals of 2 weeks (2W) and 3 weeks (3W). Each numerical figure on the abscissa (1, 2, 3, and 4) indicates the number of administration.
[FIG. 4] A graph showing the results of measurements of the tetramer positive rate in the spleen at 1 week after final administration in 2 repeats (white bar) and 3 repeats (black bar) of administration of 2 kinds or 3 kinds of memory CTL induction enhancers (anti-CD25 antibody: PC61, cyclophosphamide: CYP, TLR7 ligand: R848) and a peptide vaccine at intervals of 2 weeks.
[FIG. 5] A graph showing suppression of the tumor growth in vivo by a combination of various memory CTL induction enhancers (anti-CD25 antibody: PC61, cyclophosphamide: Chemo, TLR7 ligand: R848) and a peptide vaccine. Shown are time courses of the proliferation of EG7 after administration of PBS for white squares, administration of the peptide vaccine and R848 for black squares, and administration of a combination of the peptide vaccine and 3 kinds of memory CTL induction enhancers (anti-CD25 antibody, cyclophosphamide, R848) for white circles.
[FIG. 6] A graph showing the tetramer positive rate in the spleen at 4 days after re-administration of a peptide vaccine with the addition of various memory CTL induction enhancers ((a) TLR7 ligand: R848, TLR9 ligand: CpG, TLR3 ligand: polyAU; (b) cyclophosphamide: CYP, carmustine: CAR) or a combination thereof, or without the addition (indicated by (-)).
[FIG. 7] A graph showing the tetramer positive rate in the spleen at 4 days after re-administration of a peptide vaccine with the addition of various memory CTL induction enhancers ((b) cyclophosphamide: CYP; (c) anti-CD25 antibody: clone PC61, anti-CD152 antibody: clone 4F10, anti-GITR antibody: clone DTA1) or a combination thereof, or without the addition (indicated by (-)).
[FIG. 8] A graph showing the tetramer positive rate in the spleen at 4 days after re-administration of a peptide vaccine with the addition of a combination of various memory CTL induction enhancers ((a) TLR7 ligand: R848, TLR9 ligand: CpG, TLR3 ligand: polyAU; (b) cyclophosphamide: CYP; (c) anti-CD25 antibody: clone PC61, anti-CD152 antibody: clone 4F10, anti-GITR antibody: clone DTA1), or without the addition (indicated by (-)).

## Claims

1. A memory CTL induction enhancer comprising a combination of substance (b) with substance (a) and/or substance (c), wherein substances (a), (b) and (c) are:
(a) an activator of antigen-presenting cells,
(b) an inducer of homeostatic proliferation after induction of lymphopenia, and
(c) a suppressor of regulatory T cells.

2. The memory CTL induction enhancer of claim 1, comprising a combination of said substance (a) and said substance (b).

3. The memory CTL induction enhancer of claim 2, wherein said substance (a) and said substance (b) are administered simultaneously, separately, or sequentially.

4. The memory CTL induction enhancer of claim 2, wherein the memory CTL induction enhancer is a combination drug.

5. The memory CTL induction enhancer of claim 2, wherein the memory CTL induction enhancer is a kit comprising a drug containing said substance (a) and a drug containing said substance (b).

6. A memory CTL induction enhancer containing said substance (b), to be used in combination with a drug containing said substance (a).

7. A memory CTL induction enhancer containing said substance (a), to be used in combination with a drug containing said substance (b).

8. The memory CTL induction enhancer of any one of claims 2 to 7, wherein said substance (a) is a TLR activator.

9. The memory CTL induction enhancer of claim 8, wherein the TLR activator is a TLR7 ligand, a TLR9 ligand, or a TLR3 ligand.

10. The memory CTL induction enhancer of any one of claims 2 to 9, wherein said substance (b) is a cancer chemotherapeutic substance that induces lymphopenia.

11. The memory CTL induction enhancer of claim 10, wherein the cancer chemotherapeutic substance that induces lymphopenia is cyclophosphamide or carmustine.

12. The memory CTL induction enhancer of any one of claims 2 to 11, which is used along with an antigen protein or an antigen peptide derived from the antigen protein.

13. The memory CTL induction enhancer of any one of claims 2 to 12, to be used to treat or prevent cancer or viral infectious disease.

14. A method of enhancing the induction of memory CTL, comprising administering effective amounts of said substance (a) and said substance (b) as active components to a mammal.

15. The method of enhancing the induction of memory CTL of claim 14, wherein said substance (a) and said substance (b) are administered simultaneously, separately, or sequentially.

16. The method of enhancing the induction of memory CTL of claim 14 or 15, to be administered along with an antigen protein or an antigen peptide derived from the antigen protein.

17. The method of enhancing the induction of memory CTL of any one of claims 14 to 16, for the treatment or prophylaxis of cancer or viral infectious disease.

18. A use of said substance (a) and said substance (b) in the production of a memory CTL induction enhancer.

19. The use of claim 18, to be used simultaneously, separately, or sequentially in the enhancement of memory CTL induction.

20. The use of claim 18 or 19, to be used along with an antigen protein or an antigen peptide derived from the antigen protein in the enhancement of memory CTL induction.

21. The use of any one of claims 18 to 20, to be used to treat or prevent cancer or viral infectious disease.

22. The memory CTL induction enhancer of claim 1, comprising a combination of said substance (b) and said substance (c).

23. The memory CTL induction enhancer of claim 22, wherein said substance (b) and said substance (c) are administered simultaneously, separately, or sequentially.

24. The memory CTL induction enhancer of claim 22, wherein the memory CTL induction enhancer is a combination drug.

25. The memory CTL induction enhancer of claim 22, wherein the memory CTL induction enhancer is a kit comprising a drug containing said substance (b) and a drug containing said substance (c).

26. A memory CTL induction enhancer containing said substance (c), to be used in combination with a drug containing said substance (b).

27. A memory CTL induction enhancer containing said substance (b), to be used in combination a drug containing with said substance (c).

28. The memory CTL induction enhancer of any one of claims 22 to 27, wherein said substance (b) is a cancer chemotherapeutic substance that induces lymphopenia.

29. The memory CTL induction enhancer of claim 28, wherein the cancer chemotherapeutic substance that induces lymphopenia is cyclophosphamide or carmustine.

30. The memory CTL induction enhancer of any one of claims 22 to 29, wherein said substance (c) is anti-CD4 antibody, anti-CD25 antibody, anti-CD152 antibody or anti-GITR antibody.

31. The memory CTL induction enhancer of claim 30, wherein said substance (c) is anti-CD25 antibody.

32. The memory CTL induction enhancer of any one of claims 22 to 31, to be used along with an antigen protein or an antigen peptide derived from the antigen protein.

33. The memory CTL induction enhancer of any one of claims 22 to 32, to be used to treat or prevent cancer or viral infectious disease.

34. A method of enhancing the induction of memory CTL, comprising administering effective amounts of said substance (b) and said substance (c) as active components to a mammal.

35. The method of enhancing the induction of memory CTL of claim 34, wherein said substance (b) and said substance (c) are administered simultaneously, separately, or sequentially.

36. The method of enhancing the induction of memory CTL of claim 34 or 35, to be administered along with an antigen protein or an antigen peptide derived from the antigen protein.

37. The method of enhancing the induction of memory CTL of any one of claims 34 to 36, for the treatment or prophylaxis of cancer or viral infectious disease.

38. A use of said substance (b) and said substance (c) in the production of a memory CTL induction enhancer.

39. The use of claim 38, to be used simultaneously, separately, or sequentially in the enhancement of memory CTL induction.

40. The use of claim 38 or 39, to be used along with an antigen protein or an antigen peptide derived from the antigen protein in the enhancement of memory CTL induction.

41. The use of any one of claims 38 to 40, to be used to treat or prevent cancer or viral infectious disease.

42. The memory CTL induction enhancer of claim 1, comprising a combination of said substance (a), said substance (b) and said substance (c).

43. The memory CTL induction enhancer of claim 42, wherein said substance (a), said substance (b) and said substance (c) are administered simultaneously, separately, or sequentially.

44. The memory CTL induction enhancer of claim 42, wherein the memory CTL induction enhancer is a combination drug.

45. The memory CTL induction enhancer of claim 42, wherein the memory CTL induction enhancer is a kit comprising a drug containing said substance (a), a drug containing said substance (b), and a drug containing said substance (c).

46. A memory CTL induction enhancer containing said substance (c), to be used in combination with a drug containing said substance (a) and a drug containing said substance (b).

47. A memory CTL induction enhancer containing said substance (b), to be used in combination with a drug containing said substance (a) and a drug containing said substance (c).

48. A memory CTL induction enhancer containing said substance (a), to be used in combination with a drug containing said substance (b) and a drug containing said substance (c).

49. A memory CTL induction enhancer containing said substance (a), to be administered after administration of said substance (b) and said substance (c).

50. A memory CTL induction enhancer containing said substance (b) and/or said substance (c), to be administered before administration of said substance (a).

51. The memory CTL induction enhancer of any one of claims 42 to 50, wherein said substance (a) is a TLR activator.

52. The memory CTL induction enhancer of claim 51, wherein the TLR activator is a TLR7 ligand, a TLR9 ligand, or a TLR3 ligand.

53. The memory CTL induction enhancer of any one of claims 42 to 52, wherein said substance (b) is a cancer chemotherapeutic substance that induces lymphopenia.

54. The memory CTL induction enhancer of claim 53, wherein the cancer chemotherapeutic substance that induces lymphopenia is cyclophosphamide or carmustine.

55. The memory CTL induction enhancer of any one of claims 42 to 54, wherein said substance (c) is anti-CD4 antibody, anti-CD25 antibody, anti-CD152 antibody or anti-GITR antibody.

56. The memory CTL induction enhancer of claim 55, wherein said substance (c) is anti-CD25 antibody.

57. The memory CTL induction enhancer of any one of claims 42 to 56, to be used along with an antigen protein or an antigen peptide derived from the antigen protein.

58. The memory CTL induction enhancer of any one of claims 42 to 57, to be used to treat or prevent cancer or viral infectious disease.

59. A method of enhancing the induction of memory CTL, comprising administering effective amounts of said substance (a), said substance (b) and said substance (c) as active components to a mammal.

60. The method of enhancing the induction of memory CTL of claim 59, wherein said substance (a), said substance (b) and said substance (c) are administered simultaneously, separately, or sequentially.

61. The method of enhancing the induction of memory CTL of claim 60, wherein said substance (a) is administered after administration of said substance (b) and said substance (c).

62. The method of enhancing the induction of memory CTL of any one of claims 59 to 61, administered along with an antigen protein or an antigen peptide derived from the antigen protein.

63. The method of enhancing the induction of memory CTL of any one of claims 59 to 62, for the treatment or prophylaxis of cancer or viral infectious disease.

64. A use of said substance (a), said substance (b) and said substance (c) in the production of a memory CTL induction enhancer.

65. The use of claim 64, to be used simultaneously, separately, or sequentially in the enhancement of memory CTL induction.

66. The use of claim 65, wherein said substance (a) is administered after administration of said substance (b) and said substance (c) in the enhancement of memory CTL induction.

67. The use of any one of claims 64 to 66, to be used along with an antigen protein or an antigen peptide derived from the antigen protein in the enhancement of memory CTL induction.

68. The use of any one of claims 64 to 67, to be used to treat or prevent cancer or viral infectious disease.

69. An enhancer of memory CTL induction enhancement by (a) an activator of antigen-presenting cells and/or (c) a suppressor of regulatory T cells, containing (b) an inducer of homeostatic proliferation after induction of lymphopenia as an active component.
